# EUROPÄISCHE PATENTSCHRIFT

(11) **EP 2 459 095 B1**
(45) Veröffentlichungstag und Bekanntmachung des Hinweises auf die Patenterteilung: **02.07.2014**
(21) Anmeldenummer: 10737885.3
(22) Anmeldetag: 27.07.2010
(51) Int. Cl.: A61B 18/14, A61B 17/00, A61B 18/00

(54) **MONOPOLARE HF-CHIRURGISCHE SCHLINGEN**
MONOPOLAR HF SURGICAL NOOSES
BOUCLES DE CHIRURGIE HF MONOPOLAIRES

(30) Priorität: 28.07.2009 DE 102009036158
(43) Veröffentlichungstag der Anmeldung: 06.06.2012
(73) Patentinhaber: Farin, Günter, 72070 Tübingen (DE)
(72) Erfinder: FARIN, Günter, 72070 Tübingen (DE)
(74) Vertreter: Lohr, Georg
(86) Internationale Anmeldenummer: PCT/EP2010/060877
(87) Internationale Veröffentlichungsnummer: WO 2011/012616

(56) Entgegenhaltungen:
- WO-A1-2005/079901
- DE-A1- 2 808 546
- DE-A1- 3 626 371
- US-A1- 2009 182 324

## Beschreibung

### Technisches Gebiet

Die Erfindung betrifft hochfrequenzchirurgische (HF-chirurgische) Schlingen für endoskopisch kontrollierte Verfahren mit welchen Gewebe in Hohlorganen, insbesondere pathologische Mukosa- / Submukosa-Areale bzw. Tumoren des Gastrointestinaltrakts, umschlungen und HF-chirurgisch von der Wand des Hohlorgans für diagnostische und therapeutische Zwecke entfernt werden können.

### Stand der Technik

Maligne bzw. bösartig Tumoren, auch Karzinom genannt, des Gastrointestinaltrakts, also der Speiseröhre, des Magens und des Dickdarms, können bekanntlich Metastasen in anderen Organen verursachen und sind eine der vier häufigsten Todesursachen weltweit. Allein 1 Million Neuerkrankung und 500.000 Todesfälle werden jährlich durch maligne Tumoren des Dickdarms verursacht, wobei diese Tumoren in der Regel in der Mukosa bzw. Schleimhaut des jeweiligen Organs aus einer benignen bzw. gutartigen Veränderung der Mukosa entstehen und dann krebsartig in die unter der Mukosa befindlichen Gewebeschichten, also in die Submukosa und die Muskularis propria bzw. Muskelschicht, sowie in darin befindliche Blut- und Lymphgefäße hineinwächst. Ist ein maligner Tumor in Blut- und/oder Lymphgefäße hineingewachsen, können maligne Tumorzellen durch diese Gefäße in andere, insbesondere lebenswichtige, Organe gelangen und dort Metastasen bilden die nur schwierig oder gar nicht kurativ therapierbar sind. Da ableitende Blut- und Lymphgefäße bereits in der Submukosa beginnen, ist eine möglichst frühzeitige Diagnose und Entfernung maligner Tumoren der Mukosa die sicherste Prävention gegen seine Ausbreitung innerhalb und außerhalb der betroffenen Organwand und / oder die Metastasierung in anderen Organe.

Werden bei einer endoskopischen Inspektion des Gastrointestinaltrakts pathologisch auffällige Mukosa-Areale gefunden, dann ist eine diagnostische Untersuchung dieses Mukosa-Areal auf Karzinomanteile und deren Ausbreitung in die Blut- und Lymphgefäße enthaltene Submukosa angezeigt. Hierzu muss das betreffende Mukosa-Areal mit der darunter befindliche Submukosa möglichst en block, also in einem Stück, und komplett, also in der Tiefe möglichst nahe der Muskularis propria und in der flächigen Ausdehnung mit ausreichendem Abstand vom pathologisch auffälligen Mukosa-Areal, von der Organwand abgetrennt und zur pathohistologischen Untersuchung aus dem Gastrointestinaltrakt entfernt werden. Hierbei ist zu beachten, dass die Wand der Hohlorgane des Gastrointestinaltrakts, insbesondere die des Dickdarms bzw. Kolon mit ca. 2 mm sehr dünn ist, und insbesondere dass die normale Mukosa des Kolon nur ca. 0,3 mm und die darunter befindlichen normalen Submukosa nur ca. 0,3 bis 0,7 mm dünn ist, während die flächige Ausdehnung eines pathologisch auffälligen Mukosa-Areals mehrere Zentimeter im Durchmesser betragen kann.

Pathologische Mukosa-Submukosa-Areale können raumfordernd in das Lumen von Hohlorganen hinein wuchern, also aus Niveau der Oberfläche der normalen Mukosa herausragen und sogenannte Polypen bilden. Pathologische Mukosa-Submukosa-Areale können aber auch flächig und ohne aus dem aus Niveau der Oberfläche der normalen Mukosa herauszuragen entstehen.

Bekannte Verfahren zur endoskopisch kontrollierten Abtrennung und Entfernung pathologischer Mukosa-Submukosa-Areale bzw. Tumoren im Gastrointestinaltrakt, sind insbesondere die endoskopische Polypektomie (EPE), die endoskopische Mukosa-Resektion (EMR) und die endoskopische Submukosa-Dissektion ESD).

Gemeinsamer Zweck dieser Verfahren ist die komplette Entfernung von Tumoren mit diagnostischer und/oder therapeutischer Absicht. Dieser Zweck ist erreicht, wenn die pathohistologische Untersuchung des entfernten Gewebes die Erfüllung der Kriterien einer sog. R0-Resektion bestätigt, d.h., dass die EPE, EMR oder ESD das gesamte pathologische, insbesondere maligne Tumor-Gewebe entfernt hat, bzw. dass sie im gesunden Gewebe außerhalb des pathologischen, insbesondere außerhalb des malignen Gewebes erfolgte. Bezüglich einer pathohistologischen Untersuchung des entfernten Gewebes sowie der räumlichen Zuordnung eines insbesondere malignen Befundes im entfernten Gewebe zu der jeweiligen Ektomie-, Resektions- oder Dissektionsgrenze am betreffenden Organ ist eine Entfernung in toto und en block, d.h. insgesamt und in einem Stück, zweckmäßig und insbesondere bei großen bzw. großflächigen Tumoren gar Voraussetzung einer sicheren pathohistologischen Untersuchung und Bewertung des entfernten Gewebes bezüglich Radikalität der Entfernung benignen und insbesondere malignen Gewebe.

Mit "Endoskopischer Polypektomie (EPE)" ist allgemein ein Verfahren gemeint, bei welchem eine schlingenförmige HF-chirurgische Elektrode, allgemein Polypektomieschlinge genannt, um einen zu entfernenden Polypen geschlungen und HF-chirurgisch möglichst in toto und en block vom betreffenden Organ bzw. seiner Organwand ektomiert bzw. abgetrennt wird. Eine R0 Polypektomie in toto und en block ist nur möglich, wenn eine Polypektomieschlinge sicher um die Basis bzw. nahe der Muskularis propria der betreffenden Organwand unterhalb des zu ektomierenden Polypen geschlungen und der Polyp dann in toto und en block nahe der Muskularis propria HF-chirurgisch ektomiert werden kann.

Ragt ein pathologisches Mukosa/Submukosa-Areal zu wenig oder gar nicht über das normale Mukosa-/Submukosa-Niveau heraus, dann kann es mit einer Polypektomieschlinge nicht sicher oder gar nicht umschlungen und ektomiert werden. In diesem Fall injiziert man beispielsweise physiologische NaCl-Lösung in die Submukosa unterhalb des betreffenden Mukosa-Areals bzw. Tumors um das zu entfernende Mukosa-/Sub-mukosa-Areal so über das Niveau der normale Mukosa-/Submukosa-Niveau anzuheben, dass es mit einer Polypektomieschlinge umschlungen und HF-chirurgisch möglichst nahe der darunter befindlichen Muskularis propria der Organwand reseziert werden kann. Dieses Verfahren wird dem entsprechend, wie bereits oben erwähnt, endoskopische Mukosaresektion (EMR) genannt.

Bekannte Instrumente zur EPE sowie EMR haben im Wesentlichen eine HF-chirurgische Schlinge, einen flexiblen Katheter, mindestens einen flexiblen Manipulationsdraht, der innerhalb des Katheters in axialer Richtung zum Herausschieben und Hineinziehen der Schlinge aus bzw. in das distale Ende des Katheters sowie zum Leiten des zum HF-chirurgischen Entfernen des von der Schlinge umschlungenen Gewebes erforderlichen HF-Stroms verwendet wird, sowie einen Griff am proximalen Ende des Katheters, der aus einer Gleitschiene und einem Gleiter zum manuellen Herausschieben bzw. Hineinziehen der Schlingen aus dem bzw. in das distale Ende des Katheters besteht. Am Gleiter ist mindestens ein elektrischer Kontakt zum Anschluss eines HF-chirurgischen Generators (HF-Generator) angeordnet. Mit Polypektomieschlinge bezeichnet man allgemein pars pro toto das gesamte Instrument inklusive einer hierin integrierten Polypektomieschlinge, als auch speziell eine vom Instrument separate Polypektomieschlinge. Zur besseren Unterscheidung wird im Folgenden die separate Polypektomieschlinge kurz Schlinge, und das gesamte Instrument inklusive Schlinge kurz Instrument genannt. Instrumente mit fest integrierter Schlinge sollen mit Rücksicht auf hygienische Probleme mangels ausreichender Reinigungs- und Desinfektionsmöglichkeiten nur einmal bzw. am selben Patienten angewendet werden. Instrumente mir auswechselbarer Schlinge können ausreichend gereinigt und desinfiziert werden und dürfen folglich mehrmals bzw. an verschiedenen Patienten angewendet werden. Diese Erfindung betrifft folglich sowohl separate Schlingen als auch Instrumente mit integrierter Schlinge.

Bezüglich der Applikationstechnik der Schlinge unterscheidet man zwischen symmetrischen Schlingen bzw. Schlingen, die symmetrisch öffnen und schließen, und asymmetrischen Schlingen bzw. Schlingen, die asymmetrisch öffnen und schließen. Für die Beschreibung der Unterschiede zwischen symmetrischen und asymmetrischen Schlingen ist es zweckmäßig, die Schlingen aus zwei Schlingenabschnitten bestehend zu betrachten, die an ihren proximalen und distalen Enden zu Schlinge miteinander verbunden sind.

Monopolare symmetrische Schlingen weisen zwei Schlingenabschnitte auf, die gleich lang sind und die an ihren proximalen Enden mit dem distalen Ende eines Manipulationsdrahts verbunden sind und folglich gleichzeitig und mit derselben Geschwindigkeit aus dem distalen Ende eines Katheters heraus geschoben bzw. in das distale Ende des Katheters hineingezogen werden bzw. werden können. Folglich müssen die beiden Schlingenabschnitte gleich lang sein, unabhängig davon, ob die beiden miteinander verbundenen Schlingenabschnitte spiegelsymmetrisch gleich oder verschieden geformt bzw. vorgeformt sind.

Monopolare asymmetrische Schlingen weisen zwei Schlingenabschnitte auf, wobei einer länger als der andere ist. Nur das proximale Ende des längeren der beiden Schlingenabschnitte ist mit dem distalen Ende eines Manipulationsdrahts verbunden ist und kann somit aktiv aus dem distalen Ende eines Katheters heraus geschoben bzw. in das distale Ende des Katheters hineingezogen werden. Der kürzere Schlingenabschnitt, der an seinem distalen Ende mit dem längeren Schlingenabschnitt an dessen distalem Ende mechanisch verbunden ist, kann passiv vom längeren Schlingenabschnitt aus dem distalen Ende des Katheters bis zu einem am proximalen Ende des kürzeren Schlingenabschnitts wirkenden Anschlag einer im distalen Ende des Katheters befindlichen Anschlagvorrichtung herausgezogen werden. Bekannte Anschlagvorrichtungen sind mit einer Mitnehmervorrichtung kombiniert. Hierbei ist der längere Schlingenabschnitt mit einem mechanischen Mitnehmer ausgestattet, der bei Zurückziehen des längeren Schlingenabschnitts ab einer definierten Position den kürzeren Schlingenabschnitt in das distale Ende des Katheters mit hineinzieht. Diese kombinierte Anschlag- Mitnehmervorrichtung ist bei bekannten asymmetrischen Polypektomieschlingen direkt am distalen Ende des Katheters angeordnet.

Ein derartiges Instrument ist aus der DE 3626371 A1 bekannt.

Seit Einführung der EPE sowie der EMR besteht der Wunsch, immer größere Polypen bzw. pathologische Mukosa/Submukosa-Areale des Gastrointestinaltrakts für diagnostische und/oder therapeutische Zwecke zu entfernen, und dies mit Rücksicht auf eine pathohistologische Untersuchung, wie bereits oben erwähnt, möglichst in toto und en block. Mit der Größe der in toto und en block ektomierten Polypen bzw. resezierten Mukosa/Submukosa-Areale nehmen allerdings bei Anwendung bisher verfügbarer Schlingen für die EPE sowie für die EMR und der zum Betreiben dieser Schlingen verfügbaren HF-Generatoren auch die hieraus resultierenden Probleme und die weiter unten aufgeführten Komplikationen zu.

Eines dieser Probleme besteht darin, das insbesondere bei en block Abtrennung großer Polypen oder großflächiger Mukosa-/Submukosa-Areale, insbesondere wenn diese durch submuköse Unterspritzung noch größer werden als sie es bereits vor der Unterspritzung sind, ist die hierfür erforderliche elektrische Leistung. Da der HF-chirurgische Schneideffekt dann und nur dann entstehen kann, wenn zwischen der zum Schneiden verwendeten Schlinge und dem zu schneidenden Gewebe bei ausreichend hoher HF-Spannung elektrische Lichtbogen entstehen, die das nahe der Polypektomie- bzw. Mukosektomieschlinge befindliche Gewebe wegbrennen (Pyrolyse), muss das schlingennahe Gewebe vorher auf die Siedetemperatur von Wasser erhitzt werden damit zwischen Schlinge und Gewebe eine elektrisch isolierende Wasserdampfschicht entsteht in der dann bei ausreichender elektrischer Feldstärke elektrische Lichtbogen entstehen. Erfolgt die Erhitzung dieses Gewebes zu langsam bzw. zeitlich verzögert, was als sogenannte Anschnittverzögerung gefürchtet wird, so kann die hierbei entstehende Wärme aus dem schlingennahen Gewebe in benachbarte Gewebe diffundieren und diese thermisch schädigen. Ist die Schlingen aus oben genanntem Grunde nahe der Organwand appliziert, dann kann hierdurch die Muskularis propria thermisch geschädigt werden. Eine thermische Schädigung der Muskularis propria oder gar der Serosa eines Hohlorgans des Gastrointestinaltrakts hat in der Regel eine Perforation dieses Organs und nicht selten eine hierdurch notwendige offen chirurgische Operation zur Folge.

Für einen ausreichend verzögerungsfreien Anschnitt bei der Polypektomie bzw. Mukosaresektion sind ca. 0,5 Ampere HF-Strom pro cm Schlingenlänge erforderlich. Da HF-Generatoren von bekannten HF-Chirurgiegeräten maximal 1,5 bis 3 Ampere generieren, können nur Polypen bzw. Mukosa-/Submukosa-Areale mit einem Durchmesser im Applikationsbereich der Schlinge von maximal ca. 1 bis 2 cm - was einer effektiven Schlingenlänge von ca. 3 bis 6 cm entspricht - mit ausreichend geringer Anschnittverzögerung en block abgetrennt werden. Bei größeren Polypen bzw. Mukosa-/Submukosa-Arealen bleibt der Schneideffekt gar völlig aus.

Um dieses Problem zu lösen wird von G. Maslanka (DE OS 100 28 413 A1) vorgeschlagen, die Schlinge, dort Elektrode genannt, bzw. den Schlingendraht, dort Elektrodenkern genannt, mit einer Isolierummantelung zu beschichten. Es wird hier zumindest an einem Teilbereich der Elektrode die freie Elektrodenfläche des Elektrodenkerns durch einen eine Vielzahl von Öffnungen enthaltenden Abschnitt der Isolierummantelung reduziert. Alternativ wird die wirksame Elektrodenfläche der Schlingenelektrode durch eine Isolierummantelung der beiden Schlingenabschnitte auf einen Teilbereich in der Nähe der Elektrodenspitze begrenzt. Letzteres wurde bereits 1975 von Karl Storz (DE 2514501) und 1990 von L.F. Doll (US 5,078,716) vorgeschlagen.

Die Schlinge entsprechend DE 2514501 ist eine bipolare Schlinge, umfassend zwei spiegelsymmetrisch geformte Schlingenhälften, dort Elektroden genannt. Hier bilden die beiden Elektroden eine Schlinge, indem sie an ihren Enden mittels eines Isolierstückes miteinander verbunden sind und das die Elektroden auf ihrer ganzen Länge mit Ausnahme eines Bereichs in der Nähe des Isolierstückes mit einer Isolierschicht versehen sind.

Bipolare Schlingen haben sich in der klinischen Anwendung jedoch nicht bewährt. Ein Grund hierfür sind die elektrischen Isolationsstrecken am proximalen und / oder am distalen Ende zwischen den beiden Schlingenabschnitten. Zum HF-chirurgischen Schneiden muss die Amplitude der HF-Spannung zwischen einer zum Schneiden verwendeten aktiven Elektrode und dem zu schneidenden Gewebe mindestens 200 Volt erreichen. Da bei bipolaren Instrumenten an jeder der beiden aktiven Elektroden gleichzeitig mindestens 200 Volt mit entgegengesetzter Polarität bzw. Phasenlage erreicht werden müssen, müssen die elektrischen Isolationsstrecken zwischen den beiden Aktiven Elektroden insbesondere am proximalen und / oder am distalen Ende der Schlinge, wo der Abstände zwischen den beiden Elektroden sehr kurz sind, einer Spannungsamplitude bzw. Amplitudendifferenz von mindestens 400 V standhalten. Entstehen an diesen Stellen elektrische Lichtbogen zwischen den beiden Elektroden, dann können diese Elektroden infolge der hohen Temperatur dieser elektrischen Lichtbogen schmelzen. Bipolare Schlingen haben sich deswegen in der Praxis nicht bewährt.

In der US 5,078,716 "electrosurgical apparatus for resecting abnormal protruding growth" wird anhand von Figur 1 eine monopolare Polypektomieschlinge beschrieben, deren beide Schlingenabschnitte proximal bis auf relativ kurze Strecken an deren distalen Enden elektrisch isoliert sind, so dass nur eine relativ kurze Strecke am distalen Ende der Schlinge unisoliert und dadurch HF-chirurgisch effektiv ist. Derartige Schlingen benötigen zwar sowohl während der Anschnittphase als auch während der Schnittphase weniger HF-Strom als gleichgroße Schlingen ohne Isolation, haben jedoch den Nachteil, dass HF-chirurgisch effektive Teil der Schlinge aus der Perspektive eines Endoskops immer hinter dem Polypen, also außer Sichtkontrolle ist und das Risiko besteht, dass insbesondere die distale Spitze der Schlinge unkontrolliert die Organwand perforieren kann. Letzteres zu vermeiden ist unter anderem auch Gegenstand der DE 100 28 413 A1. So wird hier die aktive Elektrodenfläche durch eine Ummantelung mit Öffnungen reduziert. Allerdings kann diese Elektrodenform die Schnittführung behindern.

Sowohl bei elektrochirurgischen Instrumenten nach US 5,078,716, DE 100 28 413 A1 als auch bei Koagulationsistrumenten entsprechend DE 25 14 501 können die HF-chirurgisch wirksamen Elektrodenflächen nur am distalen Ende der Schlinge angeordnet werden, mit dem Nachteil, dass diese aus der Blickrichtung eines Endoskops immer hinter dem zu entfernenden Gewebe liegen und der Schnitt folglich ohne Sichtkontrolle durchgeführt werden muss, was insbesondere bei großen Polypen bzw. Mukosa-/Submukosa-Arealen riskant ist.

Ein weiteres Problem ist die Applikation der Schlinge insbesondere um große und oder bizarr geformte Polypen bzw. um durch Injektion physiologischer NaCl - Lösung oder anderer Injektionsmittel in die Submukosa, der sogenannten submukösen Unterspritzung, vergrößerte und / oder bizarr geformte Polypen oder Mukosa-/Submukosa-Areale. Um dieses Problem zu lösen, wurden und werden spezielle Schlingenformen, beispielsweise hexagonale oder crescente Schlingen, und Konstruktionen, insbesondere asymmetrische Schlingen oder rotierbare symmetrische Schlingen, entwickelt, mit welchen auch diese Polypen bzw. Mukosa/Submukosa-Areale umschlungen werden können. Allerdings dürfen alle diese Schlingen für einen der Muskularis propria nahe Abtrennung des Tumors aus oben genanntem Grunde während der HF-chirurgischen Schnittführung nicht gegen die Organwand gedrückt werden.

Ein weiteres Problem ist die Applikation und Führung der Schlinge möglichst nahe der Organwand um den Tumor aus oben genannten Gründen nahe der Muskularis propria abzutrennen. Mit multifilen gelitzten oder geflochtenen Standard Schlingen ist das in der Regel nicht praktizierbar, weil diese Schlingen hierzu zu weich bzw. zu flexibel sind Zur Beherrschung dieses Problems werden spezielle Schlingen empfohlen, wie beispielsweise monofile Schlingen oder Flachbandschlingen, die derart biegesteif sind, dass sie fest gegen die Organwand bzw. in die Mukosa gedrückt werden können. Die Anwendung dieser Schlingen ist allerdings insofern sehr riskant, als sie infolge des Drucks gegen die Organwand auch in oder gar durch die Organwand schneiden können.

Ein weiteres Problem insbesondere bei großen Polypen oder Mukosa-/Submukosa-Areale mit in der Regel glitschiger Oberfläche kann das Abrutschen der Schlinge während der Applikation bzw. Manipulation aus der beabsichtigten Applikationsstelle sein. Da weit geöffnete Schlingen beim Umschlingen insbesondere flacher Polypen bzw. Mukosa-/Submukosaareale nicht ausreichend kräftig gegen die betreffende Organwand gedrückt werden können bzw. dürfen, können sie beim Schließen der Schlinge über die glitschige Schleimhaut aus der beabsichtigten Lage abrutschen.

Zur Vermeidung dieses Problems sind Polypektomieschlingen bekannt, die mit kleinen Krallen bzw. Spornen ausgestattet sind oder bei welchen der Schlingendraht an mehreren Stellen derart gefaltet ist, dass kleine Krallen bzw. Zähne entstehen, die beim Schließen der Schlinge in das Gewebe hinein krallen bzw. hineinbeißen sollen um ein Abrutschen zu vermeiden. Diese Krallen bzw. Zähne dürfen jedoch nicht zu lang bzw. zu groß sein, weil sie sonst das Zurückziehen der Schlinge in die ca. nur 1 mm kleine Öffnung am distalen Ende des Katheters behindern. Bei asymmetrischen Schlingen stören derartige Krallen, Haken bzw. Zähne die Funktion der oben beschriebenen Mitnehmervorrichtungen.

Obwohl die endoskopische Polypektomie und die endoskopische Mukosaresektion heute als klinisch etablierte Verfahren gelten, sind diese Verfahren, vermutlich auch infolge der oben aufgeführten Probleme, mit verschiedenen Komplikationen belastet.

Diesbezügliche Komplikationen sind insbesondere Blutungen aus verletzten Blutgefäßen, Perforationen der Organwand und Rezidive infolge in kompletter Entfernung eines Tumors. Die Rate dieser Komplikationen steigt u.a. proportional mit der Größe des zu entfernen Tumors (T. Rösch: Endoskopische Mukosaresektion im oberen und unteren Gastrointestinal-Trakt. Dtsch Med Wochenschrift 2004; 129: 126-129 MUPS). Sowohl wegen der oben aufgeführten Probleme als auch wegen der dem mittleren Durchmesser des Tumors steigenden Komplikationsrate können die bisher verfügbaren oben aufgeführten Verfahren und Schlingen nur bei Polypen bzw. Mukosa/Submukosa-Areale mit einem mittleren Durchmesser bis maximal ca. 2 cm in toto und en block entfernt werden. Größere Polypen bzw. Mukosa/Submukosa-Areale können mit diesen Verfahren nur in mehreren kleineren Portionen, in sog. piecemeal Technik, und oft auch nicht radikal bzw. vollständig entfernt werden, was Rezidive zur Folge haben kann.

Um große Mukosa-/Submukosa-Areale in toto und en block möglichst nahe der Muskularis propria von Organwänden zu entfernen, die mit verfügbaren Schlingen und HF-Generatoren nicht sicher oder gar nicht in toto und en block entfernt werden können, wurden und werden verschiedene endoskopische Submukosa Dissektions (ESD) Verfahren entwickelt und einige davon bereits klinisch angewendet. Gemeinsames Merkmal dieser Verfahren ist das HF-chirurgische Abpräparieren des betreffenden Mukosa-/Submukosa-Areals von der Organwand möglichst nahe der Muskularis propria, beispielsweise mit einer Nadelelektrode. Diese Verfahren setzen jedoch eine hohe manuelle Geschicklichkeit, Erfahrung, ständiges Training, Risikobereitschaft und sehr viel Operationszeit voraus. Bisher gibt es nur wenige Experten, die diese Verfahren praktizieren.

### Darstellung der Erfindung

Es ist allgemeine Aufgabe der Erfindung, monopolare symmetrische sowie monopolare asymmetrische Polypektomieschlingen HF-chirurgische Schlingen für die endoskopisch kontrollierte Abtrennung pathologischer Gewebe, insbesondere Tumoren der Mukosa und / oder Submukosa von Organwänden des Gastrointestinaltrakts, zu entwickeln, bei deren Anwendung die oben aufgeführten Probleme und / oder Komplikationen weniger oder gar nicht vorhanden sind.

Es ist insbesondere Aufgabe der Erfindung, HF-chirurgische Schlingen zu entwickeln, mit denen auch derart große Tumoren in toto und en block von Organwänden des Gastrointestinaltrakts HF-chirurgisch abgetrennt werden können, die bisher der Piecemeal Technik, der endoskopischen Mukosa Dissektion (ESD) oder gar der offenen Chirurgie vorbehalten sind.

Es ist außerdem Aufgabe der Erfindung, diese Schlingen so zu gestalten, dass sie vor und insbesondere auch während der HF-chirurgischen Schnittführung gegen die Wand eines Hohlorgans des Gastrointestinaltrakts gedrückt werden kann ohne HF-chirurgisch in die Wand hineinzuschneiden.

Die Aufgabe wird durch eine monopolare Schlinge mit der ein zu entfernendes Gewebe umschlungen und HF-chirurgisch getrennt werden kann gelöst. Die Schlinge kann sowohl eine symmetrische als auch eine asymmetrische Schlinge sein. Sie umfasst zwei Schlingenabschnitte, die jeweils ein proximales und ein distales Ende aufweisen. Es ist wenigstens ein Schlingenabschnitt an dem proximalen Ende mit einem innerhalb eines Katheter beweglichen Manipulationsdraht verbunden oder kann zumindest mit diesem verbunden werden. Durch den Manipulationsdraht kann nun der wenigstens eine Schlingenabschnitt in den Katheter hineingezogen sowie aus diesem herausgeschoben werden. Die beiden Schlingenabschnitte sind an ihren distalen Enden miteinander verbunden oder bestehen aus einem Stück. Weiterhin ist einer der beiden Schlingenabschnitte derart elektrisch leitfähig, dass Strom aus einem HF-Chirurgiegenerator in das Gewebe, an dem dieser Schlingenabschnitt anliegt fließen kann. Der andere der beiden Schlingenabschnitte ist über seine ganze Länge derart elektrisch isoliert, dass kein Strom aus einem HF-Chirurgiegenerator in das Gewebe, an dem der Schlingenabschnitt anliegt fließen kann. Bevorzugt ist der isolierte Schlingenabschnitt mit dem Manipulationsdraht verbunden.

Besonders vorteilhaft ist es, wenn die elektrische Isolation an der Oberfläche des isolierten Schlingenabschnitts und/oder an dem distalen und/oder proximalen Ende des Schlingenabschnitts vorgesehen ist. Der isolierte Schlingenabschnitt kann aber auch aus einem Isoliermaterial bestehen.

Alternativ kann die Isolation am und/oder im Katheter und/oder am Manipulationsdraht und/oder an wenigstens einer Verbindungsstelle mit dem isolierten Schlingenabschnitt vorgesehen sein. Bevorzugt ist die Isolation zwischen dem Manipulationsdraht und dem isolierten Schlingenabschnitt. In diesen Fällen kann der isolierte Schlingenabschnitt auch aus elektrisch leitendem Material bestehen. Durch die Kombination mehrerer Isolationen kann die gesamte Isolation verbessert werden.

Vorzugsweise umfasst wenigstens einer der Schlingenabschnitte einen Draht, beispielsweise einen metallischen Runddraht und/oder Flachdraht und/oder eine Drahtlitze.

Vorzugsweise umfasst wenigstens einer der Schlingenabschnitte ein federelastisches Material.

Vorteilhafterweise bilden die beiden Schlingenabschnitte an ihren distalen Enden miteinander ein federelastisches Biegescharnier, welches ein wiederholbares federelastisches Öffnen und Schließen der Schlinge ermöglicht.

Vorzugsweise sind die beiden Schlingenabschnitte an ihren distalen Enden mechanisch und elektrisch oder nur mechanisch miteinander durch ein Verbindungselement, welches wahlweise elektrisch leitfähig oder isolierend ist, verbunden.

Eine erfindungsgemäße Schlinge kann als symmetrische Schlinge ausgeführt sein. Bei derartigen symmetrischen Schlingen, d.h. Schlingen, bei denen die beiden Schlingenabschnitte in etwa gleich lang sind, ist es grundsätzlich egal, welcher der beiden Schlingenabschnitte elektrisch isoliert ist oder aus elektrisch nicht leitfähigem Material besteht bzw. welcher der beiden Schlingenabschnitte aus elektrisch leitfähigem Material besteht und nicht elektrisch isoliert ist.

Der Vorteil einer erfindungsgemäßen symmetrischen Schlinge im Vergleich zu bekannten symmetrischen Schlingen besteht nicht nur darin, dass sie nur 50% der elektrischen Leistung entsprechender aber elektrisch nicht isolierten symmetrischer Schlingen braucht, so dass mit ihr ca. doppelt so große Tumoren en block von der Wand eines Hohlorgans abgetrennt werden können, sondern dass der elektrisch nicht isolierte bzw. HF-chirurgisch aktive Schlingenabschnitt aus der Perspektive eines Endoskops zumindest teilweise gesehen werden kann. Da die durch HF-Strom verursachten thermischen Effekte längs des gesamten HF-chirurgisch aktiven Schlingenabschnitts in der Regel gleichzeitig und weitgehend identisch entstehen, ist es bereits ein Vorteil, dass man zumindest den durch ein Endoskop sichtbaren Teil dieses Schlingenabschnittes beobachten kann.

Bei bekannten symmetrischen Schlingen, bei welchen beide Schlingenabschnitte an ihren proximalen Enden elektrisch isoliert sind, wie Beispielsweise in DE 10028413 A1 anhand der dortigen Figuren 14 bis 17, oder in US 5,078,716 anhand der dortigen Figur 1 vorgeschlagen, hat man keine Kontrolle ob und wenn nicht, warum der beabsichtigte HF-chirurgische Effekt nicht entsteht oder ob gar unbeabsichtigte thermische Effekte entstehen, die beispielweise eine thermische Schädigung der Organwand verursachen könnten, letzteres beispielsweise infolge einer zu langen Anschnittverzögerung.

Eine erfindungsgemäße Schlinge kann insbesondere eine asymmetrische Schlinge sein, bei der die beiden Schlingenabschnitte unterschiedlich lang sind, so dass ein kurzer Schlingenabschnitt kürzer als ein langer Schlingenabschnitt ist. Bei dieser Schlinge ist der lange Schlingenabschnitt elektrisch isoliert und an seinem proximalen Ende mit dem Manipulationsdraht verbunden.

Diese asymmetrische Schlinge hat im Vergleich zu bekannten asymmetrischen Schlingen mehrere Vorteile.

Ein Vorteil besteht darin, dass sie weniger als 50% der elektrischen Leistung entsprechender aber elektrisch nicht isolierten asymmetrischer Schlingen braucht. Ist der kurze, elektrisch nicht isoliert bzw. HF-chirurgisch aktive Schlingenabschnitt beispielsweise 2 cm lang und der lange, elektrisch isolierte bzw. HF-chirurgisch nicht aktive Schlingenabschnitt beispielsweise 10 cm lang, dann braucht diese asymmetrische Schlinge nur ca. 20% der elektrischen Leistung einer vergleichbaren nicht elektrisch isolierten Schlinge.

Ein weiterer Vorteil bei dieser asymmetrischen Schlinge besteht darin dass der kurze bzw. HF-chirurgisch aktive Schlingenabschnitt aus der Perspektive eines Endoskops fast vollständig gesehen werden kann.

Ein weiterer Vorteil bei dieser asymmetrischen Schlinge besteht darin dass der kurze bzw. HF-chirurgisch aktive Schlingenabschnitt unabhängig vom Umfang des abzutrennenden Gewebes bzw. Tumors immer gleich lang ist, selbstverständlich vorausgesetzt, dass der Mindestumfang mindestens doppelt so lang ist wie die Länge des kurzen Schlingenabschnitts. Folglich gibt es bei Anwendung dieser asymmetrischen Schlinge auch bei en block Abtrennungen sehr großen Tumoren keine Probleme bezüglich nicht ausreichender elektrischer Leitung, insbesondere keine gefährlichen Anschnittverzögerungen und hierdurch mögliche thermische Schädigungen einer Organwand des Gastrointestinaltrakts. Hierdurch können auch große Tumoren der Mukosa massiv submukös unterspritzt und dann en block nahe der Muskularis propria HF-chirurgisch abgetrennt werden.

Ein weiterer Vorteil dieser asymmetrischen Schlinge besteht darin, dass dank der während der gesamten Schnittphase konstanten Länge des kurzen bzw. HF-chirurgisch aktiven Schlingenabschnitts die Schnittqualität (ceteris paribus) weitgehend unabhängig von der Gesamtlänge der außerhalb des distalen Endes des Katheters befindlichen Schlinge ist.

Ein weiterer Vorteil bei dieser asymmetrischen Schlinge besteht darin, dass die Schnittqualität (ceteris paribus) von Fall zu Fall und überall reproduzierbar ist.

Ein weiterer Vorteil bei dieser asymmetrischen Schlinge besteht darin, dass der lange bzw. HF-chirurgisch nicht aktive Schlingenabschnitt auch während der HF-chirurgischen Schnittphase die Organwand berühren darf, ohne befürchten zu müssen, dass die Organwand hierdurch thermisch geschädigt wird. Dieser Vorteil ist insbesondere beim Abtrennen flacher Tumoren, wenn die Mukosa vor Applikation der Schlinge um den Tumor herum im Sinne einer Zirkumzision bzw. Umschneidung aufgeschnitten wird, so dass um den Tumor herum ein Graben entsteht in den die Schlingen nahe der Muskularis propria hineingelegt wird und hierdurch einen ausreichenden Halt findet. Hierbei ist es sehr wichtig, dass die Muskularis propria nicht thermisch geschädigt wird.

Ein weiterer Vorteil bei dieser asymmetrischen Schlinge besteht darin, dass am langen bzw. HF-chirurgisch nicht aktiven Schlingenabschnitt keine elektrischen Lichtbogen entstehen und dieser mechanisch wichtige Schlingenabschnitt folglich seine mechanischen Eigenschaften nicht thermisch negativ verändert werden.

Der kurze Schlingenabschnitt einer asymmetrischen Schlinge kann passiv mittels des langen Schlingenabschnitts bis zu einem Anschlag einer im Katheter angeordneten Anschlagvorrichtung aus den Katheter herausgezogen und mittels eines am langen Schlingenabschnitt oder am Manipulationsdraht vorhandenen Mitnehmers einer am proximalen Ende des kurzen Schlingenabschnitts vorhandenen Anschlagvorrichtung in den Katheter hineingezogen werden.

Vorzugsweise sind die Anschlagvorrichtung und der Mitnehmer innerhalb des Katheters mindestens so weit nach proximal verschoben angeordnet, dass der Mitnehmer auch bei vollständig herausgeschobenem langem Schlingenabschnitt innerhalb des distalen Endes des Katheters bleibt.

Eine vorteilhafte Weiterentwicklung der asymmetrischen Schlinge ist dadurch gekennzeichnet, dass der lange bzw. HF-chirurgisch nicht aktive Schlingenabschnitt biegesteifer als der kurze bzw. HF-chirurgische Schlingenabschnitt ist. Bevorzugt wird durch den langen biegesteiferen Schlingenabschnitt der kurze Schlingenabschnitt zwischen distalem Ende des Katheters und distalem Ende des langen biegesteiferen Schlingenabschnitts wie eine Sehne eines Schließbogens gespannt. Die unterschiedliche Biegesteifigkeit kann beispielsweise durch entsprechende Materialeigenschaften, Drahtdurchmesser und/oder auch durch Formgestaltung erreicht werden. Aufgrund der höheren Biegesteifigkeit kann die Schlinge besser als bei konventionellen Schlingen, bei denen die beiden Schlingenabschnitte aus dem gleichen Material bestehen, an das Gewebe angedrückt und weiter geöffnet werden. So kann bei biegesteiferem Material die von der Schlinge umschlossene Fläche größer sein. Weiterhin tritt hierbei die Schlinge nahezu axial aus dem Katheter aus, so dass die gesamte Schlinge nahezu lateral zur Achse des Katheters entfaltet wird.

Ein Vorteil dieser Weiterentwicklung der asymmetrischen Schlinge besteht darin, dass diese Schlinge weiter öffnet und auch dann formstabil bleibt, wenn sie weit geöffnet ist.

Eine weitere vorteilhafte Weiterentwicklung dieser asymmetrischen Schlinge ist dadurch gekennzeichnet, dass der lange bzw. HF-chirurgisch nicht aktive Schlingenabschnitt und ein an seinem proximalen Ende mit ihm verbundener Manipulationsdraht rotations- bzw. torsionssteif sind, und dass der kurze bzw. HF-chirurgisch aktive Schlingenabschnitt vorzugsweise rotationsflexibel bzw. torsionsflexibel ist. Bevorzugt werden dadurch Rotationen des Manipulationsdrahts an seinem proximalen Ende möglichst direkt und weitgehend proportional auf die Schlinge übertragen. Die unterschiedliche Rotations- bzw. Torsionssteifigkeit kann durch entsprechende Materialeigenschaften und/oder Drahtdurchmesser erreicht werden.

Bevorzugt besteht der elektrisch isolierte Schlingenabschnitt aus demselben Material besteht wie der Manipulationsdraht, beispielsweise indem der lange Schlingenabschnitt eine Verlängerung des Manipulationsdrahts ist.

Eine vorteilhafte Ausführung dieser Weiterentwicklung ist dadurch gekennzeichnet, dass der lange bzw. HF-chirurgisch nicht aktive Schlingenabschnitt und / oder der Manipulationsdraht aus NITINOL bestehen.

Ein entscheidender Vorteil der beiden o.g. Weiterentwicklungen besteht darin, dass diese asymmetrische Schlingen sowohl lateral zur axialen Richtung des Katheters und/oder durch Rotation bzw. rotatorisches Schwenken der Schlinge kontrolliert und fest gegen die Organwand gedrückt werden kann, und dies auch währende der HF-chirurgischen Schnittführung. Mit dieser asymmetrischen Schlinge können beliebig große Tumoren nahe der Muskularis propria en block abgetrennt werden, die bisher der sogenannten piecemeal Technik oder der wesentlich aufwendigeren endoskopischen Submukosa-Dissektion (ESD) vorbehalten waren.

Eine weitere Ausgestaltung der asymmetrischen Schlinge ist dadurch gekennzeichnet, dass insbesondere das distale Ende des langen Schlingenabschnitts derart vorgeformt ist, dass dieses distale Ende bereits einen kleinen Bogen bildet bevor der kurze Schlingenabschnitt bis zu seinem Anschlag aus den Katheter herausgezogen ist. Durch diese Ausgestaltung wird die zum Herausschieben des langen Schlingenabschnitts erforderliche Kraft insbesondere an der Stelle, wo der kurze Schlingenabschnitt bis zu seinem Anschlag aus den Katheter herausgezogen ist und ab wo ein weiteres Herausschieben des langen Schlingenabschnitts den kurzen Schlingenabschnitt wie die Sehne eines Schießbogens spannen soll, vermindert. Außerdem wird durch diese Ausgestaltung vermieden, dass der lange Schlingenabschnitt beim Schließen der Schlinge durch die Spannung des kurzen Schlingenabschnitts unkontrolliert in den Katheter geschoben wird. Verursacht werden die durch eine Vorformung des distalen Bereichs des langen Schlingenabschnitts vermeidbaren Phänomene beim Herausschieben des langen Schlingenabschnitts aus dem Katheter sowie beim Schließen der Schlinge durch die vektorielle Richtung der hierbei wirksamen Kräfte. Diese Phänomene entstehen umso ausgeprägter, je biegesteifer der lange Schlingenabschnitt ist.

Eine vorteilhafte Ausgestaltung dieser Weiterentwicklung ist dadurch gekennzeichnet, dass die Oberfläche des langen bzw. HF-chirurgisch nicht aktiven Schlingenabschnitts mit einer Markierung ausgestattet ist, durch welche sowohl die longitudinale Vor- und Rück-Bewegung als auch die Links- und RechtsRotation des langen Schlingenabschnitt visuell kontrollierbar ist. Diese Markierung kann beispielsweise dadurch gekennzeichnet sein, dass ein wendelartiger Streifen mit einer zur Oberfläche des langen Schlingenabschnitts kontrastierenden Farbe um den langen Schlingenabschnitt appliziert ist.

Eine vorteilhafte Ausgestaltung insbesondere der asymmetrischen Schlinge betrifft die Gestaltung der Oberfläche des elektrisch isolierten Schlingenabschnitts, welche eine Struktur aufweist, so dass ein Abrutschen der Schlinge von glitschigen Geweben gemindert oder gar verhindert wird. Vorzugsweise ist die Struktur zum Schlingenzentrum gerichtet bzw. befindet sich am Innendurchmesser des Schlingenabschnitts.

Vorzugsweise ist die Oberfläche des elektrisch isolierten Schlingenabschnitts mit kleinem scharfem und / oder spitzem Granulat beschichtet, ähnlich wie es bei Schleifpapier bzw. Sandpapier üblich ist.

Vorteilhafterweise kann auch die die Oberfläche des elektrisch isolierten Schlingenabschnitts mindestens einen Grat aufweist, der einerseits ein seitliches Abrutschen der Schlinge von glitschiger Mukosa be- oder gar verhindert und andererseits das longitudinale Gleiten des langen Schlingenabschnitts auch auf nicht glitschiger Mukosa während des Schließens der Schlinge vor und während der Schnittführung nicht behindert.

Dieser mindestens eine Grat kann vorteilhafterweise integraler Bestandteil einer elektrischen Isolationsschicht auf dem isolierten Schlingenabschnitt sein.

Ein Vorteil dieser Ausgestaltung besteht darin, dass die Schlingen beim Umschlingen eines glitschigen Tumors nicht aus der beabsichtigten Position abrutscht, andererseits aber auch bei nicht glitschigen Tumoren in longitudinaler Richtung möglichst unbehindert über die Mukosa gleiten kann.

Ein weiterer Aspekt der Erfindung, ist eine monopolare Schlinge, mit der ein zu entfernendes Gewebe umschlungen und HF-chirurgisch getrennt werden kann. Die Oberfläche, bevorzugt der zum Schlingenzentrum gerichtete Teil der Oberfläche weist wenigstens einen Grat auf, der einerseits ein seitliches Abrutschen der Schlinge von dem zu entfernenden Gewebe be- oder gar verhindert und andererseits das longitudinale Gleiten der Schlinge während des Schließens der Schlinge vor und während der Schnittführung nicht behindert oder gar verhindert.

Ein anderer Aspekt der Erfindung betrifft ein HF-chirurgisches Instrument mit einer erfindungsgemäßen monopolaren Schlinge. Das Instrument hat einen Katheter mit einem distalen und einem proximalen Ende, einen Griff für monopolare symmetrische und/oder asymmetrische Schlingen am proximalen Ende des Katheters, eine monopolare Schlinge und wenigstens einen Manipulationsdraht innerhalb des Katheters zwischen Griff und Schlinge zur Manipulation der Schlinge und zum Leiten von HF-Strom zur Schlinge.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
- Figur 1: zeigt ein Ausführungsbeispiel einer erfindungsgemäßen monopolaren symmetrisch öffnenden und schließenden Polypektomieschlinge.
- Figur 2: zeigt ein Detail des in Figur 1 dargestellten Ausführungsbeispiels
- Figur 3: zeigt ein Ausführungsbeispiel einer erfindungsgemäßen monopolaren asymmetrisch öffnenden und schließenden Polypektomieschlinge.
- Figur 4: zeigt ein Ausführungsbeispiel des Grats zur Vermeidung des Abrutschens der Schlinge
- Figur 5: zeigt einen vorgeformten Bogen im distalen Bereich des langen Schlingenabschnitts

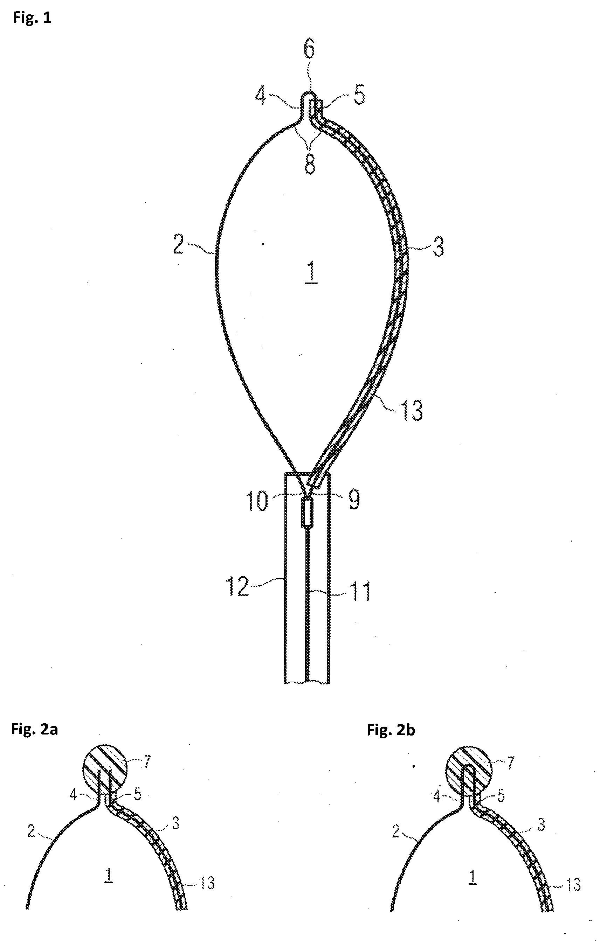

Figur 1 zeigt ein Ausführungsbeispiel einer monopolaren symmetrisch öffnenden und schließenden Schlinge 1 entsprechend der Erfindung, mit welcher ein zu entfernendes Gewebe umschlungen und HF-chirurgisch entfernt werden kann. Diese Schlinge umfasst zwei im Wesentlichen gleich lange Schlingenabschnitte 2, 3 aus federelastischem metallischem Runddraht, Flachdraht, Drahtlitze und/oder dergleichen, die an ihren distalen Enden 4, 5 direkt 6 oder durch ein in Figur 2 schematisch dargestelltes Verbindungselement 7 miteinander verbunden sind und hier ein federelastisches Biegescharnier 8 bilden, und die an ihren proximalen Ende 9, 10 mit einem Manipulationsdraht 11 verbunden sind oder verbunden werden können, der innerhalb eines Schlauchs bzw. Katheters 12 geführt ist und zum Hereinziehen in sowie zum Herausschieben der Schlinge 1 aus den Katheter 12 dient und der auch als Leiter für den zum HF-chirurgischen Entfernen biologischen Gewebes erforderlichen HF-Strom genutzt werden kann. Einer der beiden Schlingenabschnitte, hier beispielsweise der Schlingenabschnitt 3, ist vollständig mit einer elektrischen Isolation 13 isoliert und der andere Schlingenabschnitt, hier beispielsweise der Schlingenabschnitt 2, ist nicht elektrisch isoliert ist. Die elektrische Isolation 13 kann beispielsweise ein Kunststoffschlauch, insbesondere ein elektrisch isolierender flexibler Schrumpfschlauch, oder eine Kunststoffbeschichtung sein.

Figur 2a zeigt im Detail das distale Ende der Schlinge 1, wobei die distalen Enden 4, 5 der beiden Schlingenabschnitte 2, 3 durch ein Verbindungselement 7 verbunden sind, das vorzugsweise aus elektrisch isolierendem Material besteht und vorzugsweise gegen hohe Temperaturen elektrischer Lichtbogen, die beim Schneiden entstehen, resistent ist. Hierdurch kann ein Abbrand des Isolationsmaterials 13 infolge elektrischer Lichtbogen im Bereich des nicht isolierten Schlingenabschnitts 2 verhindert werden. Außerdem soll dieses Element auch verhindern, dass die Wärme aus dem distalen Ende 4 des Schlingenabschnitts 2 direkt in das distale Ende 5 des Schlingenabschnitts 3 fließen und dort das Isolationsmaterial 13 thermisch beschädigen kann. Wird ein ausreichend temperaturresistentes Material verwendet, dann kann das distale Ende der Schlinge auch wie in Figur 2b dargestellt ausgeführt werden.

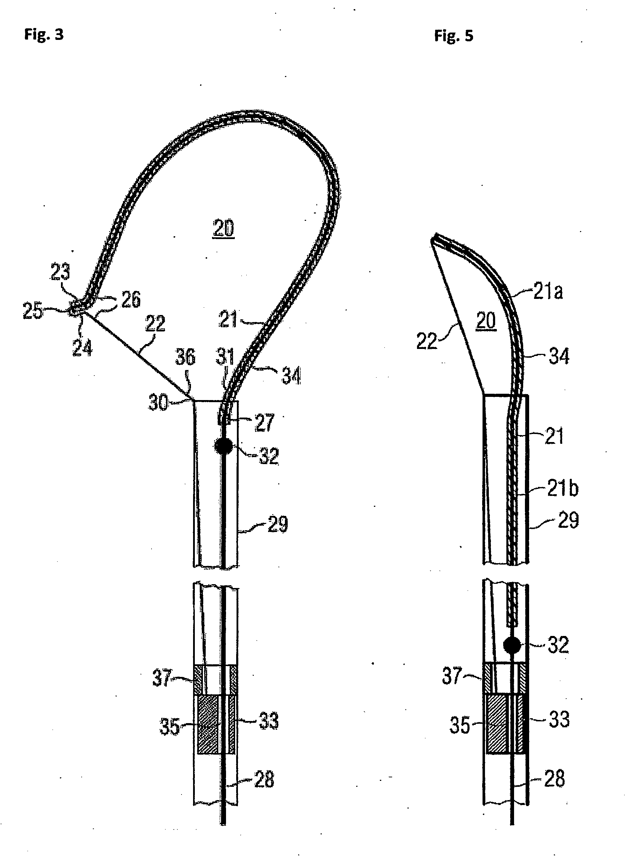

Figur 3 zeigt ein Ausführungsbeispiel einer monopolaren asymmetrisch öffnenden und schließenden Schlinge 20 entsprechend der Erfindung, mit welcher ein zu entfernendes Gewebe umschlungen und HF-chirurgisch entfernt werden kann. Diese Schlinge umfasst zwei verschieden lange Schlingenabschnitte, nämlich einen langen 21 und einen kurzen 22 aus federelastischem metallischem Runddraht, Flachdraht, Drahtlitze und/oder dergleichen, die an ihren distalen Enden 23, 24 direkt 25 oder wie in Figur 2 schematisch dargestellt, durch ein Verbindungselement 7 verbunden sind und hier ein federelastisches Biegescharnier 26 bilden. Der lange Schlingenabschnitt 21 ist an seinem proximalen Ende 27 mit einem Manipulationsdraht 28 verbunden, der innerhalb eines Schlauchs bzw. Katheters 29 geführt ist und zum aktiven Hereinziehen in sowie zum aktiven Herausschieben des langen Schlingenabschnitts 21 aus den Katheter 29 dient. Der kurze Schlingenabschnitt 22 kann passiv vom langen Schlingendraht 21 sowohl aus den Katheter 29 bis zu einem Anschlag 37 einer Anschlagvorrichtung 35, 37 herausgezogen als auch von einem Mitnehmer 32 einer Mitnehmervorrichtung 32, 33 in ihn hineingezogen oder geschoben werden. Das Prinzip der Anschlagvorrichtung sowie der Mitnehmervorrichtung ist an sich schon lange bekannt und nicht erfindungswesentlich. Erfindungswesentlich ist jedoch im Vergleich zu bekannten asymmetrischen Polypektomieschlingen, dass diese Vorrichtungen weit vom distalen Ende 30 des Katheters 29, wo diese Vorrichtungen bei bekannten asymmetrischen Polypektomieschlingen angeordnet sind, nach proximal in den Katheter 29 hinein verlagert sind, dessen Zweck anschließend und weiter unten beschrieben ist. Der lange Schlingenabschnitt 21 ist vorzugsweise längs seiner gesamten Länge mit einer elektrischen Isolation 34 überzogen oder beschichtet. Durch die Isolationsschicht 34 auf dem langen Schlingenabschnitt 21 kann der Außendurchmesser dieses Schlingenabschnitts so groß sein, dass er nicht durch das konstruktiv bedingt enge Loch 35 der Mitnehmervorrichtung 33 klemmfrei hindurch geführt werden kann. Der Mitnehmer 32 kann gleichzeitig auch Isolator sein. Der kurze Schlingenabschnitt 22 ist elektrisch nicht isoliert

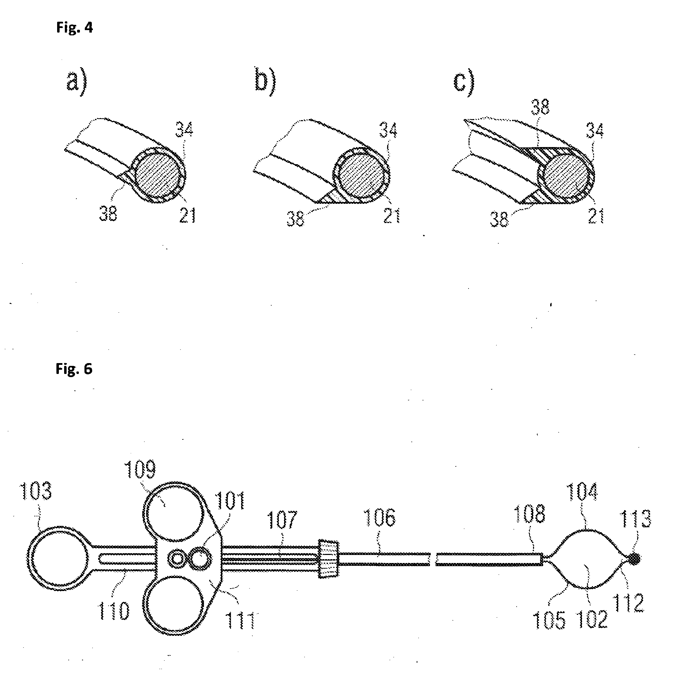

Figur 4 zeigt eine vorteilhafte Ausgestaltung insbesondere der asymmetrischen Schlinge ist dadurch gekennzeichnet, dass am Innendurchmesser des langen bzw. HF-chirurgisch nicht aktiven Schlingenabschnitts 21 mindestens ein mehr oder weniger scharfer Grat 38 angeordnet ist, der einerseits ein seitliches Abrutschen der Schlinge von glitschiger Mukosa be- oder gar verhindert und andererseits das longitudinale Gleiten des langen Schlingenabschnitts auch auf nicht glitschiger Mukosa während des Schließens der Schlinge vor und während der Schnittführung nicht behindert oder gar verhindert. Dieser mindestens eine Grat kann beispielsweise integraler Bestandteil einer elektrischen Isolationsschicht 34 auf dem langen Schlingenabschnitt 21sein. Der Grat kann beispielsweise entsprechend Fig. 5a oder Fig. 5b oder Fig. 5c gestaltet sein. Bei Asymmetrischen Schlingen entsprechend der Erfindung soll der lange bzw. HF-chirurgisch nicht aktive Schlingenabschnitt beim Schließen der Schlinge während der Applikation und während der Schnittführung nur longitudinal in sowie gegen die Zugrichtung über das Gewebe rutschen. Deswegen ist es zweckmäßig, die Oberfläche dieses Schlingenabschnitts so zu gestalten, dass der Gleitwiderstand in longitudinaler Richtung möglichst gering und in lateraler Richtung möglichst groß ist.

Figur 5 zeigt ein Ausführungsbeispiel der weiteren Ausgestaltung der asymmetrischen Schlinge bei welchem der distale Bereich 21a des langen Schlingenabschnitts 21 derart vorgeformt ist, dass dieser distale Bereich bereits einen kleinen Bogen bildet bevor der kurze Schlingenabschnitt 22 bis zu seinem Anschlag 33, 37 aus den Katheter 29 herausgezogen ist. Der nicht vorgeformte Bereich 21b des langen Schlingenabschnitts 21 befindet sich bis dahin noch im Katheter 29.

Figur 6 zeigt ein Instrument zur EPE sowie EMR. Es umfasst im Wesentlichen eine HF-chirurgische Schlinge 102 mit den beiden Abschnitten 104 und 105, die über eine direkte Verbindung 113 miteinander verbunden sind und ein federelastisches Biegeelement 112 bilden. Die Schlinge kann durch die Verriegelung 101 verriegelt werden. Das Instrument umfasst weiterhin einen flexiblen Katheter 106, mindestens einen flexiblen Manipulationsdraht 107, der innerhalb des Katheters in axialer Richtung zum Herausschieben und Hineinziehen der Schlinge aus bzw. in das distale Ende 108 des Katheters 106 sowie zum Leiten des zum HF-chirurgischen Entfernen des von der Schlinge umschlungenen Gewebes erforderlichen HF-Stroms verwendet wird, sowie einen Griff 109 am proximalen Ende des Katheters, der aus einer Gleitschiene 110 und einem Gleiter 111 zum manuellen Herausschieben bzw. Hineinziehen der Schlingen aus dem bzw. in das distale Ende des Katheters besteht. Am Gleiter ist mindestens ein elektrischer Kontakt zum Anschluss eines HF-chirurgischen Generators (HF-Generator) angeordnet.

### Bezugszeichenliste

- 1: symmetrische Schlinge
- 2: Schlingenabschnitt einer symmetrische Schlinge
- 3: Schlingenabschnitt einer symmetrische Schlinge
- 4: distales Ende der symmetrischen Schlinge
- 5: distales Ende der symmetrischen Schlinge
- 6: direkte Verbindung der distalen Enden der symmetrischen Schlinge
- 7: Verbindungselement
- 8: federelastisches Biegeelement
- 9: proximales Ende der symmetrischen Schlinge
- 10: proximales Ende der symmetrischen Schlinge
- 11: Manipulationsdraht der symmetrischen Schlinge
- 12: Katheter der symmetrischen Schlinge
- 13: elektrische Isolation
- 20: asymmetrische Schlinge
- 21: langer Schlingenabschnitt
- 21a: distaler Bereich des langen Schlingenabschnitts
- 21b: proximaler Bereich des langen Schlingenabschnitts
- 22: kurzer Schlingenabschnitt
- 23: distales Ende des langen Schlingenabschnitts
- 24: distales Ende des kurzen Schlingenabschnitts
- 25: direkte Verbindung des langen mit dem kurzen Schlingenabschnitt
- 26: federelastisches Biegescharnier
- 27: proximales Ende des langen Schlingenabschnitts
- 28: Manipulationsdraht der asymmetrischen Schlinge
- 29: Katheter der asymmetrischen Schlinge
- 30: distales Ende des Katheters der asymmetrischen Schlinge
- 31: proximales Ende der Isolationsschicht (34)
- 32: Element der Mitnehmervorrichtung (32, 33)
- 33: Element der Mitnehmer-. (32, 33) sowie der Anschlagvorrichtung. (33, 37)
- 34: Isolationsschicht des langen Schlingenabschnitts der asymmetrischen Schlinge
- 35: Loch im Element (33) der Mitnehmervorrichtung (32, 33)
- 36: proximales Ende des kurzen Schlingenabschnitts (22)
- 37: Element der Anschlagvorrichtung (33, 37)
- 38: Grat
- 101: Verriegelung
- 102: HF-chirurgische Schlinge
- 103: Proximales Ende
- 104: Schlingenabschnitt einer symmetrische Schlinge
- 105: Schlingenabschnitt einer symmetrische Schlinge
- 106: Flexibler Katheter
- 107: Flexibler Manipulationsdraht
- 108: Distales Ende
- 109: Griff
- 110: Gleitschiene
- 111: Gleiter
- 112: federelastisches Biegeelement
- 113: direkte Verbindung der distalen Enden der symmetrischen Schlinge

## Patentansprüche

1. Monopolare Schlinge (1, 20), mit der ein zu entfernendes Gewebe umschlungen und HF-chirurgisch getrennt werden kann, umfassend zwei Schlingenabschnitte (2, 3, 21, 22), wobei wenigstens ein Schlingenabschnitt an seinem proximalen Ende (9, 10, 31) mit einem Manipulationsdraht (11, 28) verbunden ist oder verbunden werden kann, mit welchem dieser Schlingenabschnitte in einen Katheter (12, 29) hineingezogen sowie aus diesem herausgeschoben werden kann und die Schlingenabschnitte an ihren distalen Enden (4, 5, 23, 24) miteinander verbunden sind oder aus einem Stück bestehen,
einer der beiden Schlingenabschnitte (2, 22) an der Oberfläche elektrisch leitfähig ist und der andere der beiden Schlingenabschnitte (3, 21) elektrisch isoliert ist,
wobei im Falle einer symmetrischen Schlinge die beiden Schlingenabschnitte (2, 3) gleich lang sind und an ihren proximalen Enden (9, 10) mit dem Manipulationsdraht (11) verbunden sind, oder
die beiden Schlingenabschnitte (21, 22) unterschiedlich lang sind, so dass ein kurzer Schlingenabschnitt (22) kürzer als ein langer Schlingenabschnitt (21) ist, wobei der lange Schlingenabschnitt (21) elektrisch isoliert und an seinem proximalen Ende (31) mit dem Manipulationsdraht (28) verbunden ist, und der kurze Schlingenabschnitt (22) passiv mittels des langen Schlingenabschnitts (21) bis zu einem Anschlag einer im Katheter (29) angeordneten Anschlagvorrichtung (33, 37) aus den Katheter herausgezogen und mittels eines am langen Schlingenabschnitt (21) oder am Manipulationdraht (28) vorhandenen Mitnehmers (32) einer am proximalen Ende des kurzen Schlingenabschnitts vorhandenen Anschlagvorrichtung (33) in den Katheter hineingezogen werden kann.

2. Monopolare Schlinge nach Anspruch 1,
**dadurch gekennzeichnet, dass**
an der Oberfläche des isolierten Schlingenabschnitts und/oder an dem distalen und/oder proximalen Ende des Schlingenabschnitts eine elektrische Isolation vorgesehen ist.

3. Monopolare Schlinge nach Anspruch 1,
**dadurch gekennzeichnet, dass**
wenigstens einer der Schlingenabschnitte (2, 3, 21, 22) einen Draht, und/oder metallischen Runddraht und/oder Flachdraht und/oder Drahtlitze umfasst.

4. Monopolare Schlinge nach Anspruch 1 oder 2,
**dadurch gekennzeichnet, dass**
wenigstens einer der Schlingenabschnitte (2, 3, 21, 22) ein federelastisches Material umfasst.

5. Monopolare Schlinge nach Anspruch 1, 2 oder 3,
**dadurch gekennzeichnet, dass**
die beiden Schlingenabschnitte (2, 3, 21, 22) an ihren distalen Enden (4, 5) miteinander ein federelastisches Biegescharnier (8, 26) bilden, welches ein wiederholbares federelastisches Öffnen und Schließen der Schlinge ermöglicht.

6. Monopolare Schlinge nach einem der vorhergehenden Ansprüche,
**dadurch gekennzeichnet, dass**
die beiden Schlingenabschnitte (2, 3, 21, 22) an ihren distalen Enden (4, 5, 23, 24) miteinander durch ein Verbindungselement (7), welches wahlweise elektrisch leitfähig oder isolierend ist, verbunden sind.

7. Monopolare Schlinge nach einem der vorhergehenden Ansprüche,
**dadurch gekennzeichnet, dass**
die Anschlagvorrichtung (33, 37) und der Mitnehmer (32) innerhalb des Katheters (29) mindestens so weit nach proximal verschoben angeordnet sind, dass der Mitnehmer (32) auch bei vollständig herausgeschobenem langem Schlingenabschnitt (21) innerhalb des distalen Endes des Katheters (29) bleibt.

8. Monopolare Schlinge nach einem der vorhergehenden Ansprüche,
**dadurch gekennzeichnet, dass**
der elektrisch isolierte Schlingenabschnitt (21) biegesteifer und/oder torsionssteifer als der andere Schlingenabschnitt (22) ist.

9. Monopolare Schlinge nach einem der vorhergehenden Ansprüche,
**dadurch gekennzeichnet, dass**
der elektrisch isolierte Schlingenabschnitt (3, 21) aus demselben Material besteht wie der Manipulationsdraht (28) und/oder wenigstens einer der Schlingenabschnitte (2, 3, 21, 22) und/oder der Manipulationsdraht (28) aus NITINOL bestehen.

10. Monopolare Schlinge nach einem der vorhergehenden Ansprüche,
**dadurch gekennzeichnet, dass**
der elektrisch isolierte Schlingenabschnitt (3, 21) auf seiner Oberfläche eine Markierung hat, an welcher Bewegungen dieses Schlingenabschnitts visuell erkennbar sind.

11. Monopolare Schlinge nach einem der vorhergehenden Ansprüche,
**dadurch gekennzeichnet, dass**
der elektrisch isolierte Schlingenabschnitt (3, 21) in einem distalen Bereich (21a) derart vorgeformt ist, dass dieser Bereich bereits einen kleinen Bogen bildet bevor der kurze HF-chirurgisch aktivierbare Schlingenabschnitt (22) bis zu seinem Anschlag (33, 37) aus den Katheter (29) herausgezogen ist.

12. Monopolare Schlinge nach einem der vorhergehenden Ansprüche,
**dadurch gekennzeichnet, dass**
die Oberfläche des elektrisch isolierten Schlingenabschnitts (3, 21) eine Struktur aufweist, und/oder mit einem scharfem und / oder spitzem Granulat beschichtet ist, und/oder einen Grat aufweist so dass ein Abrutschen der Schlinge von glitschigen Geweben gemindert oder gar verhindert wird.

13. Monopolare Schlinge (1) nach einem der vorhergehenden Ansprüche, mit der ein zu entfernendes Gewebe umschlungen und HF-chirurgisch getrennt werden kann, deren Oberfläche wenigstens einen Grat aufweist, der einerseits ein seitliches Abrutschen der Schlinge von dem zu entfernenden Gewebe be- oder gar verhindert und andererseits das longitudinale Gleiten der Schlinge während des Schließens der Schlinge vor und während der Schnittführung nicht behindert oder gar verhindert.

14. HF-chirurgisches Instrument umfassend
- einen Katheter mit einem distalen und einem proximalen Ende
- einen Griff für monopolare symmetrische und/oder asymmetrische Schlingen am proximalen Ende des Katheters
- eine Schlinge nach einem der vorhergehenden Ansprüche am distalen Ende des Katheters und
- einen Manipulationsdraht innerhalb des Katheters zwischen Griff und Schlinge zur Manipulation der Schlinge und zum Leiten von HF-Strom zur Schlinge.

## Claims

1. Monopolar snare (1, 20), with which tissue to be removed can be ensnared and RF-surgically removed, including two snare sections (2, 3, 21, 22), wherein at least one snare section at its proximal end (9, 10, 31) is or can be connected to a manipulation wire (11, 28) with which this snare section can be pulled into as well as pushed out of a catheter (12, 29) and the snare sections are connected to each other at their distal ends (4, 5, 23, 24) or are made in one piece,
one of the two snare sections (2, 22) is electrically conductive at the surface and the other of the two snare sections (3, 21) is electrically insulated,
wherein in case of a symmetrical snare, both snare sections (2, 3) are of equal length and are connected at their proximal ends (9, 10) to the manipulation wire (11), or
the two snare sections (21, 22) are of different length, so that a short snare section (22) is shorter than a longer snare section (21), wherein the long snare section (21) is electrically insulated and connected at its proximal end (31) to the manipulation wire (28), and the short snare section (22) can be passively pulled out of the catheter by means of the long snare section (21) as far as the stop of a stop device (33, 37) disposed in the catheter (29) and can be pulled into the catheter by means of a driver (32) at the long snare section (21) or at the manipulation wire (28) of a stop device (33) at the proximal end of the short snare section,

2. Monopolar snare according to claim 1,
**characterized in that**
an electrical insulation is provided at the surface of the insulated snare section and/or at the distal end and/or the proximal end of the snare section.

3. Monopolar snare according to claim 1,
wherein **characterized in that**
at least one of the snare sections (2, 3, 21, 22) comprises a wire and/or metallic round wire and/or flat wire and/or wire strand.

4. Monopolar snare according to claim 1, ,
wherein **characterized in that**
wherein at least one of the snare sections (2, 3, 21, 22) incorporates spring-elastic material.

5. Monopolar snare according to claim 1, 2, or 3,
**characterized in that**
the two snare sections (2, 3, 21, 22) at their distal ends (4, 5) together form a spring-elastic hinge (8, 26) which enables a repeatable spring-elastic opening and closing of the snare.

6. Monopolar snare according to one of the preceding claims,
**characterized in that**
the two snare sections (2, 3, 21, 22) are at their distal ends (4, 5, 23, 24) connected together by means of a connecting element (7) which is optionally electrically conductive or insulating.

7. Monopolar snare accordingto one of the preceding claims,
**characterized in that**
the stop device (33, 37) and the driver (32) are disposed inside the catheter (29) displaced at least so far in a proximal direction that the driver (32) remains inside the distal end of the catheter (29) even when the long snare section (21) has been completely pulled out.

8. Monopolar snare according to one of the preceding claims,
**characterized in that**
the electrically insulated snare section (21) is more bending resistant and/or torsionally more rigid than the other snare section (22).

9. Monopolar snare according to one of the preceding claims,
**characterized in that**
the electrically insulated snare section (3, 21) is made of the same material as the manipulation wire (28), and/or at least one of the snare sections (2, 3, 21, 22) and/or the manipulation wire (28) comprise of NITINOL.

10. Monopolar snare according to one of the preceding claims,
**characterized in that**
the electrically insulated snare section (3, 21) has a mark on its surface which enables movements of this snare section to be visually detected.

11. Monopolar snare according to one of the preceding claims,
**characterized in that**
the electrically insulated snare section (3, 21) is in a distal area (21a) preshaped so that this area already forms a small curve before the short RF-surgically activatable snare section (22) has been pulled out of the catheter (29) to its stop (33, 37).

12. Monopolar snare according to one of the preceding claims,
**characterized in that**
the surface of the electrically insulated snare section (3, 21) has a structure, and/or is coated with a sharp and/or pointed granular material, and/or has a projecting section, so as to hinder or even prevent the snare section from slipping off slippery tissue.

13. Monopolar snare (1) according to one of the preceding claims, with which tissue to be removed can be ensnared and RF-surgically removed and the surface of which includes at least one projecting sections which hinders or even prevents the snare from laterally slipping off the tissue to be removed and, on the other, does not hinder or even prevent the snare from sliding longitudinally while the snare is closing before and during incision.

14. RF-surgical instrument including
- a catheter with a distal and a proximal end,
- a handle for monopolar symmetrical and/or asymmetrical snares at the proximal end of the catheter,
- a snare according to any one of the preceding claims at the distal end of the catheter, and
- a manipulation wire inside the catheter between the handle and the snare for manipulating the snare and conducting RF current to the snare.

## Revendications

1. Boucle monopolaire (1, 20) avec laquelle un tissu à exciser peut être entouré et sectionné par chirurgie HF, comprenant deux parties de boucle (2, 3, 21, 22), dans laquelle au moins une partie de boucle est ou peut être reliée à son extrémité proximale (9, 10, 31) à un fil de manipulation (11, 28) avec lequel cette partie de boucle peut être tirée et introduire dans un cathéter (12, 29) et sortie de celui-ci et les parties de boucle sont reliées entre elles ou se composant d'une seule pièce à leurs extrémités distales (4, 5, 23, 24),
une des deux parties de boucle (2, 22) est conductrice électrique en surface et l'autre des deux parties de boucle (3, 21) est isolée électriquement,
dans laquelle, dans le cas où la boucle est symétrique, les deux parties de boucle (2, 3) ont la même longueur et sont reliées à leurs extrémités proximales (9,10) avec le fil de manipulation (11) ou les deux parties de boucle (21, 22) ont une longueur différente, de sorte qu'une partie de boucle courte (22) est plus courte qu'une partie de boucle longue (21), la partie de boucle longue (21) étant isolée électriquement et reliée à son extrémité proximale (31) au fil de manipulation (28), et la partie de boucle courte (22) pouvant être tirée hors du cathéter passivement au moyen de la partie de boucle longue (21) jusqu une butée d'un dispositif de butée (33, 37) présent dans le cathéter (29) et pouvant être tirée dans le cathéter au moyen d'un entraîneur (32) d'un dispositif de butée (33) présent à l'extrémité proximale de la partie de boucle courte, lequel entraîneur se trouve sur la partie de boucle longue (21) ou sur le fil de manipulation (28).

2. Boucle monopolaire selon la revendication 1, **caractérisée en ce qu'**une isolation électrique est prévue sur la surface de la partie de boucle isolée et/ou sur l'extrémité distale et/ou proximale de la partie de boucle.

3. Boucle monopolaire selon la revendication 1, **caractérisée en ce que** l'une au moins des parties de boucle (2, 3, 21, 22) comprend un fil et/ou un fil rond métallique et/ou un fil plat et/ou une tresse de fil.

4. Boucle monopolaire selon la revendication 1 ou 2, **caractérisée en ce que** l'une au moins des parties de boucle (2, 3, 21, 22) comprend un matériau élastique faisant ressort.

5. Boucle monopolaire selon la revendication 1, 2 ou 3, **caractérisée en ce que** les deux parties de boucle (2, 3, 21, 22) forment ensemble à leurs extrémités distales (4, 5) une charnière flexible (8, 26) élastique à la manière d'un ressort, qui permet une ouverture et une fermeture élastiques comme un ressort et pouvant être répétées de la boucle.

6. Boucle monopolaire selon l'une des revendications précédentes, **caractérisée en ce que** les deux parties de boucle (2, 3, 21, 22) sont reliées entre elles à leurs extrémités distales (4, 5, 23, 24) par un élément de liaison (7) qui peut être au choix conducteur ou isolant électrique.

7. Boucle monopolaire selon l'une des revendications précédentes, **caractérisée en ce que** le dispositif de butée (33, 37) et l'entraîneur (32) sont repoussés dans le sens proximal à l'intérieur du cathéter (29) au moins assez loin pour que l'entraîneur (32) reste à l'intérieur de l'extrémité distale du cathéter (29), même quand la partie de boucle longue (21) est complètement sortie.

8. Boucle monopolaire selon l'une des revendications précédentes, **caractérisée en ce que** la partie de boucle isolée électriquement (21) est plus rigide en flexion et/ou en torsion que l'autre partie de boucle (22).

9. Boucle monopolaire selon l'une des revendications précédentes, **caractérisée en ce que** la partie de boucle isolée électriquement (3, 21) se compose du même matériau que le fil de manipulation (28) et/ou au moins une des parties de boucle (2, 3, 21, 22) et/ou le fil de manipulation (28) se composent de Nitinol.

10. Boucle monopolaire selon l'une des revendications précédentes, **caractérisée en ce que** la partie de boucle isolée électriquement (3, 21) possède sur sa surface un marquage qui permet de reconnaître visuellement les mouvements de cette partie de boucle.

11. Boucle monopolaire selon l'une des revendications précédentes, **caractérisée en ce que** la partie de boucle isolée électriquement (3, 21) est préformée dans une zone distale (21a) de telle manière que cette zone forme déjà un petit arc de cercle avant que la partie de boucle courte (22) pouvant être activée en chirurgie HF soit sortie du cathéter (29) jusqu'à sa butée (33, 37).

12. Boucle monopolaire selon l'une des revendications précédentes, **caractérisée en ce que** la surface de la partie de boucle isolée électriquement (3, 21) présente une structure et/ou est recouverte de granulés coupants et/ou pointus et/ou présente une barbe de façon à réduire ou même à empêcher le glissement de la boucle sur des tissus glissants.

13. Boucle monopolaire (1) selon l'une des revendications précédentes, avec laquelle un tissu à exciser peut être entouré et sectionné par chirurgie HF, dont la surface présente au moins une barbe qui réduit voire empêche un glissement latéral de la boucle par rapport au tissu à exciser et ne gêne ou n'empêche pas le glissement longitudinal de la boucle pendant sa fermeture avant et pendant la coupe.

14. Instrument de chirurgie HF comprenant
- un cathéter avec une extrémité distale et une extrémité proximale
- une poignée pour des boucles monopolaires symétriques et/ou asymétriques à l'extrémité proximale du cathéter
- une boucle selon l'une des revendications précédentes à l'extrémité distale du cathéter et un fil de manipulation dans le cathéter entre la poignée et la boucle pour la manipulation de la boucle et pour la conduction de courant HF vers la boucle.
